# EUROPÄISCHE PATENTSCHRIFT

(11) **EP 1 514 115 B1**
(45) Veröffentlichungstag und Bekanntmachung des Hinweises auf die Patenterteilung: **18.04.2007**
(21) Anmeldenummer: 03755964.8
(22) Anmeldetag: 30.05.2003
(51) Int. Cl.: G01N 33/574, C12Q 1/68, A61K 31/44

(54) **DIAGNOSTIKUM UND NACHWEISVERFAHREN FÜR EIN KARZINOM UND MITTEL ZU SEINER BEHANDLUNG**
DIAGNOSTIC AGENT, METHOD FOR DETECTING A CARCINOMA, AND MEANS FOR THE TREATMENT THEREOF
PROCEDE DE DIAGNOSTIC ET D'IDENTIFICATION D'UN CARCINOME ET AGENT POUR LE TRAITER

(30) Priorität: 31.05.2002 DE 10224534
(43) Veröffentlichungstag der Anmeldung: 16.03.2005
(73) Patentinhaber: DOLDERER, Jürgen, 65375 Oestrich-Winkel (DE)
(72) Erfinder: DOLDERER, Jürgen, 65375 Oestrich-Winkel (DE)
(74) Vertreter: Meyer-Dulheuer, Karl-Hermann
(86) Internationale Anmeldenummer: PCT/EP2003/005710
(87) Internationale Veröffentlichungsnummer: WO 2003/102588

(56) Entgegenhaltungen:
- WO-A-00/22001
- WO-A-99/54463
- SMITH GARTH A M ET AL: "Functional up-regulation of HERG K+ channels in neoplastic hematopoietic cells" JOURNAL OF BIOLOGICAL CHEMISTRY, Bd. 277, Nr. 21, 24. Mai 2002 (2002-05-24), Seiten 18528-18534, XP002260223 ISSN: 0021-9258

## Beschreibung

Die Erfindung betrifft ein Nachweisverfahren des colorektalen Karzinoms (Dickdarmkrebs) in einer Gewebeprobe des menschlichen Dick- oder Enddarms sowie ein Mittel zur Behandlung des colorektalen Karzinoms.

Karzinomerkrankungen haben bekanntlich in allen Ländern eine immer größere Bedeutung erlangt. Die Behandlung von Karzinomen und ein Therapieerfolg hängen in entscheidender Weise davon ab, dass das Karzinom rechtzeitig erkannt wird. Deshalb besteht ein großer Bedarf an zuverlässigen Karzinom-Diagnostika, insbesondere solchen, die den Nachweis von Metastasen und Mikrometastasen auch dann erlauben, wenn ein eindeutiger histologischer Befund noch nicht vorliegt oder der histologische Befund negativ ausfällt.

Das colorektale Karzinom ist die zweithäufigste Todesursache bei Krebsleiden, welches kontinuierlich in seiner Inzidenz zunimmt und häufig nach einer kurativen chirurgischen Operation wieder auftritt. Colorektale Karzinome (bösartige Dick- und Enddarmtumore) treten in den Industrieländern immer häufiger auf und stellen bei Männern das zweithäufigste und bei Frauen das dritthäufigste Krebsleiden dar. Das colorektale Karzinom macht 15% der bösartigen Neubildungen aus. Es kommen über 200.000 neue Fälle von colorektalen Karzinomen im Jahr vor, wobei über 100.000 Patienten daran versterben. Das colorektale Karzinom steht somit an zweiter Stelle der Todesursachen bei Krebsleiden.

Ein colorektales Karzinom kann de novo oder im Rahmen der Adenom-Karzinom-Sequenz in einem adenomatösen Polypen entstehen. Die Wahrscheinlichkeit einer malignen Entartung bei Adenomen liegt zwischen 1 bis 40%. Insofern stellen Patienten mit colorektalen Polypen eine Risikogruppe dar. Aus diesem Grund ist die Früherkennung von colorektalen Karzinomen in Adenomen und die zuverlässige Differenzierung zum gutartigen colorektalen Gewebe besonders für die Prognose und den Therapieverlauf von entscheidender Bedeutung.

Die Diagnose und Prognose für diese Krebsart wird durch eine Vielzahl von Eigenschaften, die zur Zeit der Erstdiagnose vorhanden sind, beeinflußt. Diese Faktoren beinhalten Alter, Geschlecht, Dauer der Symptome, Zustand der Darmobstruktion, Tumorlokalisation, Notwendigkeit von Bluttransfusion und die Qualität der chirurgischen Intervention. Bisher haben zwar eine Anzahl von Tumoreigenschaften, wie vaskuläre lymphatische Invasivität, Differenzierungsgrad und präoperative Titer von konventionellen Tumormarkern einen prognostischen Wert gezeigt, jedoch gibt es keine geeigneten Marker für die Detektion von Frühstadien des Karzinoms (Entartung von gutartigen colorektalen Vorstufen (Adenome)) oder für histopathologisch unauffällige Mikrometastasen (minimal residual disease), die der Grund für das Wiederauftreten des Karzinoms, sogar nach kurativer chirurgischer Resektion, sein können. Die bisher verwendeten Turmormarker CEA, CK 19 und CK 20 sind für die derzeitige Prognose richtungsweisend, jedoch unzuverlässig in der Diagnosedifferenzierung.

Der Erfindung liegt daher die Aufgabe zugrunde, ein Nachweisverfahren für ein colorektales Karzinom zur Verfügung zu stellen und weiterhin die Verwendung eines wirksamen Mittels zur Behandlung eines colorektalen Karzinoms zur Verfügung zu stellen.

Zur Lösung dieser Aufgabe sieht die Erfindung vor, dass eine Gewebeprobe des menschlichen Dickdarms oder Enddarms auf das Vorhandensein von HERG-Kaliumkanälen untersucht wird.

Gegenstand der Erfindung ist deshalb ein Verfahren zum Nachweis eines colorektalen Karzinoms, mit dem man die Anwesenheit mindestens eines HERG-Kaliumkanals in einer Gewebeprobe des menschlichen Dickdarms oder Enddarms, die beim gesunden Menschen frei von HERG-Kaliumkanälen ist, oder in Körperflüssigkeiten nachweist.
Der Nachweis eines HERG-Kaliumkanals mit dem erfindungsgemäßen Diagnostikum ist selbstverständlich nur dann ein sicherer Hinweis auf das Vorliegen eines colorektalen Karzinoms, wenn der Nachweis in einer Gewebeprobe des menschlichen Dick- oder Enddarms oder in Lymphknoten oder in einer Körperflüssigkeit vorgenommen wird, die beim gesunden Menschen frei von HERG-Kaliumkanälen sind. Das trifft nicht zu für das Herzmuskelgewebe und das Gehirn, in denen beim gesunden Menschen stets HERG-Kaliumkanäle vorkommen. Gelingt jedoch der Nachweis eines HERG-Kaliumkanals z.B. in einer Gewebeprobe des Dick- oder Enddarmes, in der bekanntlich normalerweise niemals HERG-Kaliumkanäle vorkommen, dann ist das ein sicherer Nachweis für das Vorliegen eines Karzinoms.

Der Karzinomnachweis für das Auffinden von HERG-Kaliumkanälen ist auch dann zuverlässig, wenn dieser Nachweis in einer Körperflüssigkeit, wie Blut, Blutplasma, Blutserum, Harn, Schweiß oder Tränenflüssigkeit oder auch im Stuhl gelingt, in denen normalerweise niemals HERG-Kaliumkanäle aufzufinden sind.

Ein Beispiel für das erfindungsgemäße Verfahren und Diagnostizieren eines Karzinoms besteht darin, dass man ein colorektales Karzinom diagnositiziert, in dem man in einer Gewebeprobe des menschlichen Dickdarmes mindestens einen HERG-Kaliumkanal nachweist.

Zur Diagnose wird eine Gewebeprobe des Dick- bzw. Enddarmes im Labor untersucht, wobei der HERG-Kaliumkanal in colorektalen Karzinomzellen exprimiert wird, der sowohl durch die hochsensitive RT-PCR-Methode als auch durch die in der Klinik verbreitete Immunhistochemie nachgewiesen werden kann. Die überraschende und wertvolle Erkenntnis besteht darin, dass der HERG-Kaliumkanal ein hochselektiver funktioneller Tumormarker für das colorektale Karzinom ist und somit in der Diagnostik, vor allen zur Erkennung von Frühstadien des Karzinoms, zum Nachweis von unauffälligen Mikrometastasen und zur Differenzierung vom gesunden colorektalen Gewebe eingesetzt werden kann.

Mitglieder der ether á go-go (EAG) Kaliumkanalfamilie, insbesondere das eagabhängige (eag-related-gen; ERG) Genprodukt, wie der humane ERG (HERG) Kaliumkanal sind zur Diagnose geeignet. Sie konnten in verschiedenen Tumorzelllinien unterschiedlicher Histogenese gefunden werden, wobei bekannt ist, dass sie bei der Zellproliferation und Transformation eine Rolle spielen.

Die reverse Transkriptase Polymerase Kettenreaktion (reverse transcriptase polymerase chain reaktion, RT-PCR) ist eine sehr effiziente und hoch sensitive Methode zum Nachweis von minimaler tumor-assoziierter mRNA Transcription. Die Immunhistochemie ist in der Klinik eine gebräuchliche und weit verbreitete Methode und daher leicht in den Routineablauf zu integrieren.

Der HERG (human eag related gene) Kaliumkanal stellt einen besonderen Typ von humanem Kaliumkanal dar, der zu der eag (ether a-go-go) Familie der spannungsaktivierten Kaliumkanäle gehört. In neoplastischen Zellen unterschiedlicher Histogenese spielt der HERG- Kaliumkanal offensichtlich eine wichtige, pathophysiologische Rolle bei den Regulationsmechanismen und bewirkt ein unlimitiertes Tumorwachstum.

Zur Behandlung von colorektalen Karzinomzellen wird erfindungsgemäß die Verwendung des Antiarrhythmikum E-4031 vorgeschlagen. Dies beruht auf der wertvollen Erkenntnis, dass der funktionelle HERG-Kaliumkanal, der für die Zellproliferation und das Karzinomwachstum eine wichtige Rolle spielt, selektiv durch das Antiarrhythmikum E-4031 blockiert werden kann. Somit sind neue Ansätze für die Krebstherapie möglich.

Das Antiarrhythmikum E-4031 ist ein 4-[1-[2-(6-Methyl-2-pyridinyl)ethyl-4-piperidinyl]carbonyl]methansulfoanilid, 2HCl der Formel

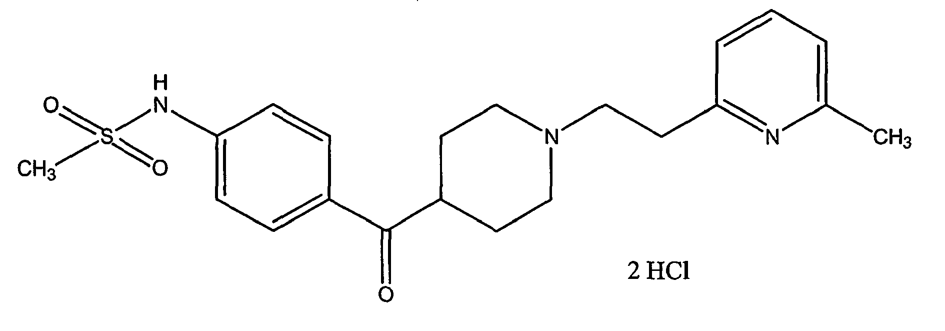

Der Erfindung liegen folgende Untersuchungen und Maßnahmen zugrunde.

### Patienten

Es wurden 24 Gewebeproben von 18 verschiedenen Patienten (12 Frauen, 6 Männer; im Alter von 51-86 Jahren, Durchschnittsalter 67,7 Jahre) mit colorektalem Karzinom der UICC Klassifikation von I-IV untersucht. Fünf Karzinome waren im Colon, vier im sigmoidalen Colon und neun im Rektum lokalisiert. Die Gewebeproben wurden von dem umgebenden Gewebe freipräpariert, um eine Überkreuzkontamination zu vermeiden. Für jede einzelne Gewebeprobe wurde ein neues Skalpel benützt.

Die klinische und histopathologische UICC sowie Dukes Klassifikation von Patienten mit colorektalem Adenokarzinom ergab folgendes:

| | |
|---|---|
| Stadium (stage) I - Dukes A : 3 | (pT1 = 1, pT2 = 2) |
| Stadium (stage) II - Dukes B : 5 | (pT3 = 5, pT4 = 0) |
| Stadium (stage) III - Dukes C : 7 | (pN1 = 4, pN2 = 3) |
| Stadium (stage) IV - Dukes D : 3 | (pM1 = 3) |

Histopathologisch negative Proben von Colongewebe von 7 Patienten (4 Frauen, 3 Männer; im Alter von 60-68 Jahren, Durchschnittsalter 62,8 Jahre), wovon 3 Proben ein tubulo-villöses Adenom des Colons und 4 Proben eine Sigmadivertikulitis waren, dienten als Negativkontrolle (Tabelle 2).

Die histopathologische Diagnosen, die UICC und Dukes Klassifikation sowie TNM Klassifikation wurden unter standardisierten Bedingungen durchgeführt. Die Gewebeprobe wurde sofort nach der Exzision in flüssigem Stickstoff tief gefroren und bei -80°C bis zur RNA Extraktion gelagert.

Die gesamte zelluläre RNA-Isolierung wird von den gefrorenen Gewebeproben mit dem Qiagen Rneasy mini-kit nach Anleitung des Herstellers (Hilden, Deutschland) durchgeführt. Der Gehalt und Reinheit der RNA wird spektrophotometrisch bei einer Wellenlänge von 260 nm und 280 nm bestimmt.

Die colorektale Karzinomzelllinie (Colo-205) wurde für die Detektion der Sensitivität dieser Experimente als positive Kontrolle verwendet.

### Reverse Transkription

Die cDNA wurde aus 2µg gesamter RNA in einem Volumen von 20µl Reaktionsmischung synthetisiert, welche 4µl von 5 x Reaktionspuffer (50 mmol/L Tris-HCl, pH 8.3, 75 mmol/L KCl und 3 mmol/L MgCl₂), 500 µmol/L dNTP, 100 µmol/L Lösung von poly-dT15-Primer (Roche Diagnostic, Mannheim, Deutschland) und 400 Einheiten von Superscript II (Gibco BRL, Gaithersburg, MD, USA) enthält. Die Mischung wurde bei 42°C für 60 min inkubiert, für 2 min auf 90°C erhitzt und dann auf Eis abgekühlt.

### Primersequenzen für die RT-PCR Untersuchung:

Folgende Primersequenzen wurden für die anschließenden RT-PCR Untersuchungen verwendet:
Die Primersequenzen für die CEA mRNA waren: A, 5'-TCTGGAACTTCTCCTGGTTCTCTCAGCTGG-3' für die outer sense; B, 5'-TGTAGCTGTTGCAAATGCTTTAAGGAAGAA-3' für die antisense; und C, 5'-GGGCCACTGTCGGCATCATGATTGG-3' für die inner sense cases .
Die Primersequenzen für die CK-19 mRNA waren: A, 5'-GTGGAGGTGGATTCCGCTCC-3' für die outer sense; B, 5'-TGGCAATCTCCTGCTCCAG-3' für die outer antisense; C, 5'-. ATGGCCGAGCAGAACCGGAA-3' für die inner sense; and D, 5'-CCATGAGCCGCTGGTACTCC-3' für die inner antisense cases.
Die Primersequenzen für die CK-20 mRNA waren: A, 5'-GCGTTTATGGGGGTGCTGGAG-3' für die outer sense; B, 5'-AAGGCTCTGGGAGGTGCGTCTC-3' für die outer antisense; C, 5'-CGGCGGGGACCTGTTTGT-3' für die inner sense; und D, 5'-CAGTGTTGCCCAGATGCTTGTG-3' für die inner antisense cases.
Die Primersequenzen für die HERG mRNA waren:
   A, primer up 5'-AGCTGATCGGGCTGCTGAAGACTG -3' und
   B, primer down 5'-AATGAGCATGACGCAGATGGAGAAG-3' .

Um die Integrität der extrahierten RNA zu untersuchen und um sicherzustellen, dass equimolare RNA verwendet wurde, wurde die extrahierte RNA mit Glyceraldehyd-3-Phosphat- Dehydrogenase (GAPDH) mittels RT-PCR kontrolliert. Die Primersequenzen für GAPDH waren: 5'-CCACCCATGGCAAATTCCATGGCA-3' sense und 5'-TCTAGACGGCAGGTCAGGTCCACC-3' antisense primers.

### Die reverse Transkriptase-Polymerase Kettenreaktion (RT-PCR):

Die zweistufige RT-PCR wurde für die Amplifikation von cDNA von CEA, von CK-19 und CK-20 und von HERG verwendet.

Die PCR wurde wie folgt ausgeführt: Die PCR wurde in einem Volumen von 50µl mit 2 µg der gesamten RNA pro Probe durchgeführt. Für die erste PCR-Runde, 2 µL Aliquots der cDNA Lösung wurden mit 10,5 µL der PCR Reaktionsmischung, welche 1,25 µL 10 x PCR Puffer (10 mmol/L Tris-HCl, pH 8.3, 50 mmol/L KCl, und 1.5 mmol/L MgCl2), 200 µmol/L dNTP, 0,5 µmol/L von jedem Primer, und 2,5 Einheiten von Platinum Taq Polymerase (Gibco BRL, Gaithersburg, MD) enhält, gemischt.

Die Reaktion wurde in einem PCR Thermocycler (Biometra, Göttingen, Germany) fortgesetzt. Für die CEA, CK-19 and CK-20 Amplifikation wurden folgende Bedingungen angewandt: Aktivierung der Taq Polymerase bei 95°C für 4 min, Template Denaturierung bei 95°C für 45 sec, Annealing bei 60°C für 45 sec und Elongation bei 72°C für 45 sec für 20 Zyklen. Die HERG-Amplifikation wurde bei 95°C, 4 min; 55°C, 1 min; 72°C, 1 min und 95°C, 1 min für 30 Zyklen durchgeführt. Zwei Mikroliter des ersten PCR Produktes wurde in ein zweites Röhrchen transferiert und weiter amplifiziert (CEA, CK-19 und CK-20: bei 95°C, 4 min; 95°C, 45 sec; 60°C, 45 sec und 72°C, 45 sec in 20 Zyklen und HERG bei 95°C, 4 min; 55°C, 1 min; 72°C, 1 min und 95°C, 1 min in 30 Zyklen). Alle Pipetierarbeiten wurden auf Eis und unter einer sterilen Werkbank durchgeführt und dann die Röhrchen in den Thermocycler gegeben. Zuvor wurden sie auf die Denaturierungstemperatur erhitzt bevor die Amplifikation begann.

Die amplifizierten DNA Fragmente waren 132 Basenpaare (bp) für CEA-, 328 Basenpaare für CK-19-, 485 Basenpaare für CK-20- und 386 Basenpaare für HERG-Primerpaare. Jede Gewebeprobe wurde mindestens zweimal untersucht.

Um die Integrität für cDNA zu überprüfen, wurden in 25 Zyklen die Amplifikation für GAPDH mit 603 Basenpaaren der cDNA Fragmente (95°C, 4min; 95°C, 45 sec; 60°C, 45 sec; 72°C, 45 sec) durchgeführt.

Die PCR Produkte wurden auf ein 2% iges Agarosegel aufgetragen und mit Ethidiumbromid gefärbt.

### Sensitivitätstests der RT-PCR-Methode

Die colorektale Karzinomzelllinie (Colo-205) diente für die Detektionssensitivität in diesen Experimenten als positive Kontrolle. Um die Sensitivität der Methode bestimmen zu können, wurden Verdünnungsreihen von 10⁶ bis zu 10⁰ der colorektalen Karzinomzellen (Colo-205) angefertigt, denen dann 10⁷ Lymphozyten von gesunden Spendern zugegeben wurden. Nach der Extraktion der gesamten RNA wurden die RT-PCR durchgeführt. Die Verdünnungsreihen der RNA zeigten, dass die Primer in der Lage waren, eine Menge mRNA, welche äquivalent zu einer Tumorzelle in 10⁶ Lymphozyten ist, zu detektieren.

### Immunhistochemie

Proben von colorektalen Karzinomen und gesundem colorektalem Gewebe wurden in gepuffertem Formalin fixiert und in Paraffin eingebettet. Die Histologie wurde an Hematoxylin und Eosin gefärbten Schnitten evaluiert. Weitere Schnitte wurden für den immunhistochemischen Nachweis verwendet.

Für die Immunhistochemie des Anti-HERG-Antikörpers wurde ein Protokoll der ABC (Avidin-Biotin-Peroxidasecomplex)-Nachweismethode verwendet.

Nachdem die Schnitte auf Objektträger aufgebracht und über Nacht bei 37°C luftgetrocknet wurden, folgte eine Deparaffinierung durch Xylol und eine Rehydrierung über eine absteigende Alkoholreihe. Die endogene Peroxidase wurde durch Immersion der Schnitte für 10 min in einer Lösung von 19 ml Phosphatpuffer, 1 ml Methanol und 200 µl 30%iges H₂O₂ blockiert. Anschließend wurden die Schnitte 6x 10 min mit PBS gespült.

Um die nicht-spezifische Antikörperbindung zu reduzieren, wurden die Schnitte mit Normalserum der Ziege vorinkubiert.

Danach erfolgte die Inkubierung mit dem primären Antikörper Rabbit Anti-ERG1 (HERG) (AB5222-200UL, Chemicon, Temecula, CA, USA) in einer Verdünnung von 1:500 für 1 h bei 4°C. Nachdem 3x mit PBS gespült wurde, sind die Schnitte in biotinyliertem Anti-Rabbit-Antikörper (Vector) in einer Verdünnung von 1:200 für 1 h bei 25°C inkubiert worden. Anschließend wurden die Schnitte 3x mit PBS gespült. Danach folgte die Inkubierung mit dem ABC Komplex (Vector) für 1 h bei 25°C. Nach mehrmaligem Spülen wurden die Schnitte für die Visualisierung des primären Antikörpers mit 3,3'-Diaminobenzidintetrahydrochlorid (DAB) (Sigma) für 3 min bei 25°C nach einem Standardprotokoll (0,05 % DAB in 0,1 M Phosphatpuffer und 0,3 % H₂O₂) behandelt.

Die Färbung der Schnitte wurde unter dem Mikroskop verfolgt. Die Färbereaktion wurde durch Spülen mit PBS gestoppt.

Bei positiver Antikörper-Reaktion wird das betroffene Gewebeareal homogen braun angefärbt.

Nach der Immunhistochemie wurden die Schnitte über Nacht getrocknet, dehydriert und anschließend eingedeckt.

### Negativ Kontrollen: gleiche Prozedur ohne Primärantikörper

Positive Kontrollen: Experimente von Anti-ERG1 (HERG)-Antikörper wurden an Schnitten des humanen Herzens durchgeführt.

### Proliferationstest der colorektalen Karzinomzelllinie Colo-205 mit E-4031

Die Proliferationsversuche (Wachstumsversuche) wurden mit dem 3-(4,5-dimethylthiazol-2-yl)-2,5-diphenyl-2H-tetrazolium-bromid MTT-Test durchgeführt. Die Zellen der colorektale Karzinomzelllinie Colo205 wurden mit 0,05% EDTA geerntet und anschließend ausgezählt. Die Zellen wurden dann in eine 96-Lochplatte mit je 10⁴ lebenden Zellen pro Loch ausgesät und 24 h zum Anhaften belassen. Nach 24 h wurde das Medium mit E-4031 in verschiedenen Konzentrationen von 1 µM, 5 µM und 10 µM ausgetauscht. Das Endvolumen war 200 µl pro Loch. Nach der Inkubation von 0 bis 7 Tagen wurden 20 µl MTT (5mg/ml in PBS) hinzugefügt und für 2 h in 37°C inkubiert. Das Medium wurde dann entfernt und 100 µl Dimethylsulfoxid (Sigma, Deutschland) wurde in jedes Loch für 10 Minuten gegeben.

Ein 96 Loch Mikrotiterplattenleser (Comtek, Deutschland) wurde für die Auswertung benutzt. Bei einer Wellenlänge von 570 nm Angstrom wurde der MTT-Test in dem ELISA- Mikrotiterplattenleser abgelesen. Die durchschnittliche Konzentration wurde in jedem Set von fünf Löchern gemessen.

Die Absorption der unbehandelten Kontrollen wurden als 100 % angenommen und dementsprechend der IC₅₀ berechnet.

### Statistische Auswertung

Ein Unterschied von P<0,05 wurde als signifikant angenommen.

### Ergebnisse der RT-PCR

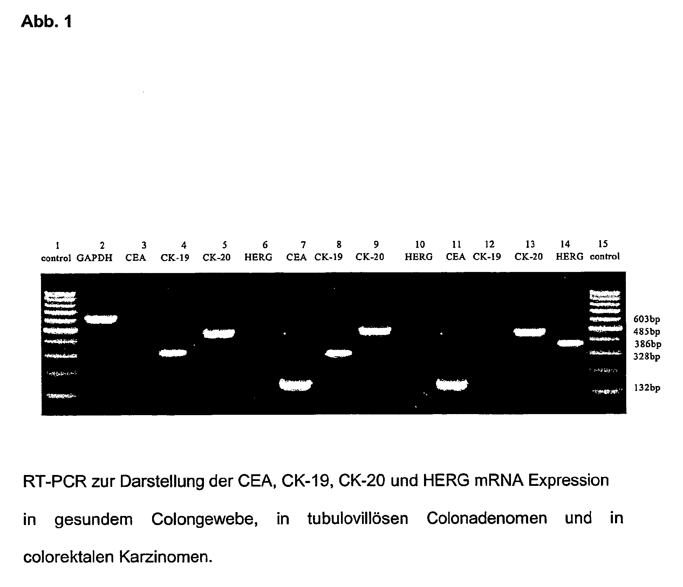

CEA, CK-19 und CK-20 wurden im Karzinom und im Kontrollgewebe sowie in der colorektalen Zellinie Colo-205 exprimiert. Die HERG Expression war im Gegenteil zu 100 % nur auf das Karzinomgewebe und die Colo-205-Zellen beschränkt. Wie in Abbildung 1 und Tabelle 1 dargestellt, zeigen Gewebeproben, die in der histopathologischen Untersuchung eindeutig ein colorektales Karzinom ergeben, eine Expression von allen Markern (CEA, CK-19, CK-20 und HERG). Colorektales Gewebe von Patienten ohne eine maligne Erkrankung exprimierte auch CEA, CK-19 und/oder CK-20, jedoch ließ sich HERG nie nachweisen (Tabelle 2). Wenn die RT-PCR ohne RT Enzyme durchgeführt wurde, ließen sich in der Zelllinie Colo-205 keine Banden nachweisen, was beweist, dass keine DNA vorhanden ist, und somit die RT-PCR Bedingungen sehr gut abgestimmt sind.

In Abbildung 1 sind in Spalten 1 und 15 100 Basenpaarstufen als Kontrollbanden dargestellt. In Spalte 2 ist die GAPDH Kontrolle zu sehen, die in allen Proben positiv war. Die Ergebnisse in den Spalten 3 bis 6 sind von einer Gewebeprobe eines Patienten mit Sigmadivertikulitis (Patient Nr. 6 in Tabelle 2) und in den Spalten 7 bis 10 von einer Gewebeprobe eines Patienten mit einem tubulovillösen Colonadenom (Patient Nr. 1 in Tabelle 2). Die Banden in den Spalten 11 bis 14 repräsentieren eine Gewebeprobe eines Patienten mit einem colorektalen Karzinom (Patient Nr. 3 in Tabelle 1). DNA Fragmente wurden auf einem 2%igen Agarosegel aufgetragen und mit Ethidiumbromid gefärbt.

Wie in Tabelle 1 zusammengefasst, konnte CEA in 22 (91.7 %), CK-19 in 19 (79.2 %), CK-20 in 23 (95.8 %), und HERG exklusiv zu 100 % in allen der 24 Gewebeproben von colorektalen Karzinomen von 18 verschiedenen untersuchten Patienten nachgewiesen werden.

In allen Proben, außer in der CK-19 und CK-20 Expression im Patienten Nr. 2, waren keine Unterschiede in den mRNA Resultaten der verschiedenen Gewebeproben von dem gleichen Untersuchungspräparat (Patient Nr. 4, 6, 7, 8, 9) vorhanden (Tabelle 1).

**Tabelle 1 Detektion von mRNA Expression der Tumormarker CEA, CK-19, CK-20 und HERG mittels RT-PCR in colorektalen Karzinomen**

| **Pat.** | **GAPDH** | **CEA** | **CK-19** | **CK-20** | **HERG** | **Staging - TNM** | **Lokalisation** |
|---|---|---|---|---|---|---|---|
| | | | | | | | |
| 1 | pos | Pos | pos | pos | **pos** | III - T4,N1(2/10),M0 | Sigmoid |
| | | | | | | | |
| 2a | pos | Pos | neg | neg | **pos** | III-T3,N1(1/9),M0 | Colon |
| 2b | pos | Pos | pos | pos | **pos** | | |
| | | | | | | | |
| 3 | pos | Pos | neg | pos | **pos** | II - T3,N0(0/15),M0 | Colon |
| | | | | | | | |
| 4a | pos | Pos | pos | pos | **pos** | IV - T3,N2(25/27),M1 | Rektum |
| 4b | pos | Pos | pos | pos | **pos** | | |
| | | | | | | | |
| 5 | pos | Neg | pos | pos | **pos** | I - T2,N0(0/6),M0 | Sigmoid |
| | | | | | | | |
| 6a | pos | Pos | pos | pos | **pos** | IV - T3,N2(11/20),M1 | Rektum |
| 6b | pos | Pos | pos | pos | **pos** | | |
| | | | | | | | |
| 7a | pos | Pos | neg | pos | **pos** | III- T2,N1(2/12),M0 | Rektum |
| 7b | pos | Pos | neg | pos | **pos** | | |
| | | | | | | | |
| 8a | pos | Pos | pos | pos | **pos** | III - T3,N1(1/35),M0 | Colon |
| 8b | pos | Pos | pos | pos | **pos** | | |
| | | | | | | | |
| 9a | pos | Pos | pos | pos | **pos** | III - T3,N2(21/27),M0 | Rektum |
| 9b | pos | Pos | pos | pos | **pos** | | |
| 10 | pos | Pos | pos | pos | **pos** | I - T2,N0(0/10),M0 | Rektum |
| 11 | pos | Pos | pos | pos | **pos** | II - T3,N0(0/4),M0 | Colon |
| 12 | pos | Pos | pos | pos | **pos** | IV - T3,N2(6/16),M1 | Sigmoid |
| 13 | pos | Pos | pos | pos | **pos** | II - T3,N0(0/7),M0 | Rektum |
| 14 | pos | Pos | pos | pos | **pos** | II - T3,N0(0/13),M0 | Colon |
| 15 | pos | Pos | pos | pos | **pos** | III - T3,N2(6/11),M0 | Rektum |
| 16 | pos | Pos | pos | pos | **pos** | I - T1,N0(0/16),M0 | Sigmoid |
| 17 | pos | pos | pos | pos | **pos** | III - T3,N2(6/36),M0 | Rektum |
| 18 | pos | neg | neg | pos | **pos** | II - T3,N0(0/16),M0 | Rektum |

Das Grading (histopathologischer Differenzierungsgrad) der colorektalen Karzinome war in allen Gewebeproben G2 außer für die Probe des Patienten Nr. 1 (G2-3), Nr. 4 (G3) und Nr. 9 (G2-3) zu erkennen.

HERG mRNA wurde in keinem (0 %) der histopathologisch negativen colorektalen Gewebeproben der 3 Patienten mit einem tubulovillösen Adenom des Colons und der 4 Patienten mit Sigmadivertikulitis, die als Negativkontrollen dienten, nachgewiesen (Abbildung 1 und Tabelle 2).

Die mRNA der Tumormarker CEA, CK-19 und CK-20 waren in allen Patienten mit einem Colonadenom positiv (100 %). In den Gewebeproben der Patienten mit Sigmadivertikulitis waren jeweils die mRNA der Marker CEA, CK-19 und CK-20 in 2 Patienten (50 %) positiv.

**Tabelle 2: Detektion von mRNA Expression der Tumormarker CEA, CK-19, CK-20 und HERG mittels RT-PCR in histopathologisch negativem colorektalem Gewebe.**

| **Pat.** | **GAPDH** | **CEA** | **CK-19** | **CK-20** | **HERG** | **Histologie** | **Lokalisation** |
|---|---|---|---|---|---|---|---|
| 1 | pos | pos | Pos | pos | **neg** | tubulovillöses Adenom | Colon |
| 2 | pos | pos | Pos | pos | **neg** | tubulovillöses Adenom | Colon |
| 3 | pos | pos | Pos | pos | **neg** | tubulovillöses Adenom | Colon |
| | | | | | | | |
| 4 | pos | pos | Neg | neg | **neg** | Sigmadivertikulitis | Sigmoid |
| 5 | pos | pos | Pos | pos | **neg** | Sigmadivertikulitis | Sigmoid |
| 6 | pos | neg | Pos | pos | **neg** | Sigmadivertikulitis | Sigmoid |
| 7 | pos | neg | Neg | neg | **neg** | Sigmadivertikulitis | Sigmoid |

### Ergebnisse der Immunhistochemie

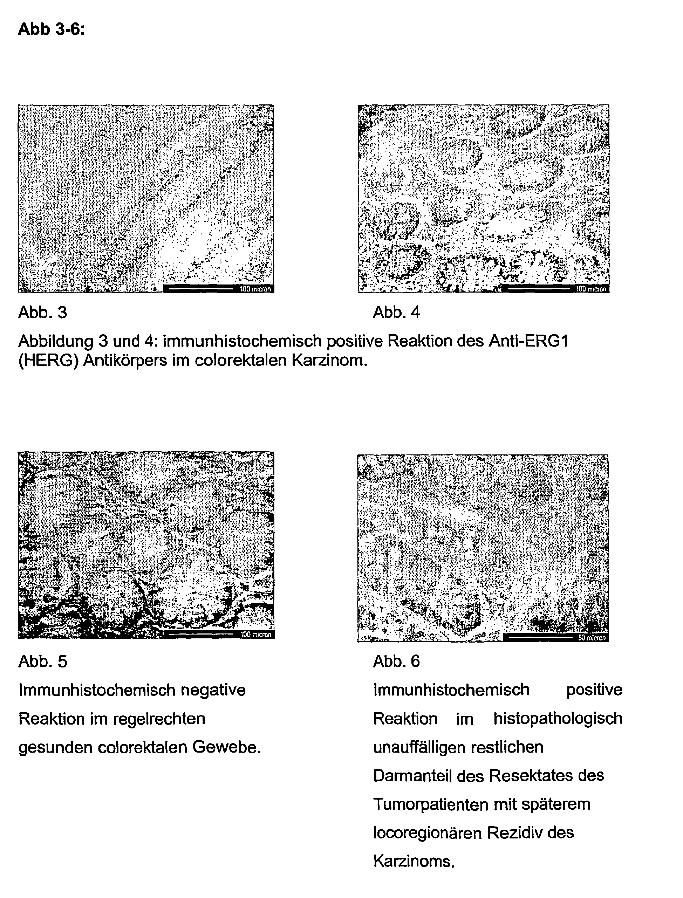

Die Ergebnisse des immunhistochemischen Nachweises des Anti-ERG1 (HERG) Antikörpers zeigten deutlich eine immunhistochemisch positive Reaktion durch eine Färbung der betroffenen, maligne entarteten Gewebe(drüsen)-anteile in allen Proben der colorektalen Karzinome (Tabelle 3). Die Immunreaktivität ist beschränkt auf den basalen Anteil des Zytoplasmas der Epithelzellen (Abbildung 3 und 4). Neben den Epithelzellen findet man diffuse Reaktionen in der Lamina propria und in den Gefäßen (Blutzellen). Weiterhin findet man in den Muskelschichten vereinzelte, nicht näher charakterisierbare Zellen.

Die histopathologisch negativen restlichen Darmanteile des Resektates derselben Tumorpatienten waren bei den meisten Gewebeproben immunhistochemisch negativ. Jedoch ließ sich bei den immunhistochemisch positiven Gewebeproben in den histopathologisch negativen restlichen Darmanteilen des Resektates vereinzelt eine schwache, aber eindeutig positive lmmunreaktion in den basalen Epithelzellen am Grund der Drüsen nachweisen (Abbildung 6). In einer repräsentativen Anzahl von Patienten wurde in einem Zeitraum von zwei Jahren nach der operativen Resektion des colorektalen Karzinoms stets ein locoregionäres Rezidiv (Wiederauftreten) des Karzinoms festgestellt (Patienten Nr. 19-25 in Tabelle 3).

Diese sensitive und zuverlässige immunhistochemische Detektion von HERG in histopathologisch noch unauffälligem Gewebe und somit der Nachweis von Mikrometastasen macht HERG zu einem zuverlässigen Tumormarker.

Die histopathologisch negativen colorektalen Gewebeproben von benignen Darmveränderungen (Sigmadivertikulitis, colorektale Adenome) sowie die gesunden colorektalen Gewebeproben (regelrechte gesunde Darmschleimhaut) zeigten in allen Fällen keine Färbung und somit eine immunhistochemisch negative Reaktion (Tabelle 4 und Abbildung 5).

**Tabelle 3: Darstellung der immunhistochemischen Ergebnisse in dem colorektalen Karzinomgewebe sowie in dem histopathologisch negativen restlichen colorektalen Gewebe desselben Tumorpatienten. (Unter den Patienten mit den durchlaufenden Nr. 1-25 sind die Patienten Nr. 1-18 der PCR enthalten.)**

| **Pat.** | **Karzinomgewebe** | **Restliche Darmanteile des Resektates histologisch negativ** |
|---|---|---|
| 1 -18 | Pos | neg |
| 19 - 25 | Pos | pos |

**Tabelle 4: Darstellung der immunhistochemischen Ergebnisse in histopathologisch negativen Geweben. (Unter den Patienten mit den durchlaufenden Nr. 1-25 sind die Patienten mit den Nr. 1-7 der PCR enthalten.)**

| **Pat.** | **Regelrechte gesunde Darmschleimhaut** |
|---|---|
| 1-6 | neg |
| | **Sigmadivertikulititen** |
| 7-18 | neg |
| | **Colorektale Adenome** |
| 19-25 | neg |

### Ergebnisse des Proliferationsassays

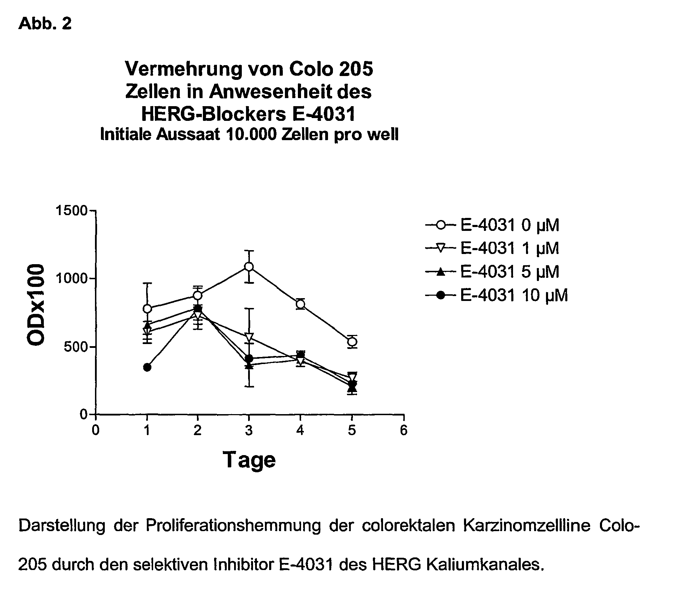

Abbildung 2 zeigt die Proliferationshemmung der colorektalen Karzinomzellline Colo-205 durch den selektiven Inhibitor E-4031 des HERG Kaliumkanales.

E-4031 wurde in 3 verschiedenen Konzentrationen (1, 5 und 10 µM) zu den Zellen hinzu gegeben und dann an 5 darauf folgenden Tagen das Wachstumsverhalten der colorektalen Karzinomzellen gemessen. Die Karzinomzellen wurden durch die Konzentrationen von E-4031 in ihrem Wachstum, durch die Blockierung des für das Karzinomwachstum wichtigen HERG-Kaliumkanals, gehemmt und starben ab.

Durch die vorliegende Erfindung konnte somit ein Tumormarker aufgefunden werden, der verlässlich kanzerogenes (bösartiges) von nicht-kanzerogenem (gutartigem) colorektalem Gewebe unterscheiden und dadurch detektieren und des Weiteren karzinogene Tumorzellen in histopathologisch unauffälligem Gewebe nachweisen kann. Dies ist für die zuverlässige Früherkennung von Karzinomzellen in colorektalen Adenomen sowie für die sichere Erkennung von Mikrometastasen im histopathologisch unauffälligen Gewebe und in den Lymphknoten für den Therapieverlauf und Prognose von ganz besonderer Bedeutung.

CEA eignet sich nicht als ein solcher Marker, da CEA mRNA nicht in colorektalen Karzinomen im Stadium I detektiert wurde, aber in allen Gewebeproben der tubulovillösen Colonadenomen und in zwei Proben der Sigmadivertikulitis, die als Negativkontrollen fungierten, exprimiert wird. Im Einklang mit den vorgelegten Ergebnissen zeigte Bhatnagar et al. mittels quantitativer Enzymimmunoassay und Immunozytochemie, dass CEA in moderat bis sehr wenig differenzierten colorektalen Tumoren gar nicht vorhanden ist. Boucher et al. demonstriert, dass CEA nicht nur in Adenokarzinomen des Colons, sondern auch in gesundem angrenzendem Schleimhautgewebe des Colons vorkommt.

CK-19 and CK-20 sind nicht nur im Serum, sondern auch im colorektalen Gewebe keine verlässlichen Tumormarker. Sie lassen sich nicht in fortgeschrittenen Karzinomen im Stadium III, jedoch in einigen Kontrollengeweben, nachweisen. Dies bestätigt frühere Beobachtungen, dass CK-19 und CK-20 auch in gesunder Darmschleimhaut gefunden werden kann, aber auch in einigen Tumoren nicht vorhanden sind. Die Beobachtung, dass das reichliche Vorkommen von CK-19 in gut differenziertem Tumorgewebe und geringgradiger Dysplasie mit geringer Zellproliferation, als auch das geringe Vorkommen in gering differenziertem Karzinomgewebe und hochgradiger Dysplasie mit einer großen Proliferationsrate, sowie die geringe Expression von CK-20 in colorektalen Karzinomen im Vergleich zu gesundem Colongewebe lässt vermuten, dass die negative Tumorprobe des Stadiums III sich in einem hohen Proliferationsstatus befindet. Die konträren Ergebnisse von der Gewebeprobe des Patienten Nr. 2 (Tabelle 1) zeigen zusätzlich, dass die Marker nicht gleichmäßig in dem Tumor verteilt sind.

Im Gegensatz zu den Ergebnissen von CEA, CK-19 und CK-20, war eine HERG-Expression in allen colorektalen Karzinomen, unabhängig vom Tumorstadium, selektiv vorhanden und in jedem Kontrollgewebe negativ. Dies ließ sich sowohl auf molekularbiologischer Ebene durch den Nachweis der HERG-RNA mit der PCR Methode als auch in der Immunhistochemie durch den Nachweis des HERG-Proteins selektiv in den colorektalen Karzinomzellen nachweisen.

In einigen histopathologisch negativen restlichen Darmanteilen des Resektates der Tumorpatienten konnte immunhistochemisch HERG positive Reaktionen nachgewiesen werden. Diese Patienten erlitten in den nachfolgenden zwei Jahren nach der operativen Resektion des Karzinoms ein locoregionäres Rezidiv des colorektalen Karzinoms. Dies ist ein Nachweis von Mikrometastasen im histopathologisch unauffälligem colorektalem Gewebe.

Durch den Nachweis von HERG mit zwei voneinander unabhängigen, sensitiven Methoden, die beide üblich sind, gewinnt die Detektionsqualität und Zuverlässigkeit enorm.

Daher ist der HERG-Kaliumkanal ein hochselektiver Tumormarker für das colorektale Gewebe.

Er ist zudem ein wertvoller Marker für andere epitheliale Karzinome, da er auch im Endometriumkarzinom vorkommt. Die vorgelegten Daten unterstützen die kürzlich auf Basis von in in-vitro Studien vermutete starke Involvierung der EAG-Kaliumkanäle, den HERG Kaliumkanal mit eingeschlossen, in die Karzinogenese. Diese Kaliumkanäle sind in verschiedenen Tumorzelllinien gefunden worden, die einen transformierten Phänotyp in transfizierten Zellen von Säugetieren übertragen und Tumorprogression in immunsupprimierten Mäusen favorisieren. Da der HERG-Kaliumkanal für das Tumorwachstum und/oder das Überleben des Tumors von immenser Bedeutung ist, ließ sich in den Proliferationsstudien nachweisen, dass durch die selektive Blockade des HERG-Kaliumkanals durch E-4031 die Karzinomzellen zum Absterben gelangen. Der HERG-Kaliumkanal, als ein funktioneller Tumormarker für das colorektale Karzinom, stellt somit nicht nur einen selektiven Tumormarker dar, sondern ermöglicht auch durch die selektive Hemmung von E-4031 einen neuen Ansatz für die Krebstherapie.

Die überraschenden und wertvollen Erkenntnisse der Erfindung lassen sich dahingehend zusammenfassen, dass eine sichere Diagnose von colorektalen Karzinomzellen durch selektive HERG-Genexpression in zwei voneinander unabhängigen Testverfahren (Immunhiostochemie und PCR) möglich ist, wobei ein neuartiger und hochselektiver Marker in Gestalt des HERG-Kaliumkanals Verwendung findet und dass sich das Wachstum der Tumorzellen durch das Antiarrhythmikum E-4031 hemmen lässt.

## Patentansprüche

1. Verfahren zum Diagnostizieren eines colorektalen Karzinoms, **dadurch gekennzeichnet, dass** man in einer Gewebeprobe des menschlichen Dickdarms oder Enddarms oder in Lymphknoten oder in Körperflüssigkeiten oder im Stuhl mindestens einen HERG-Kaliumkanal als einen selektiven Tumormarker für das colorektale Karzinom nachweist.

2. Verwendung des Antiarrhythmikums 4-[1-[2-(6-Methyl-2-pyridinyl)ethyl-4-piperidinyl]carbonyl]methansulfoanilid, 2HCl (E-4031) der Formel I 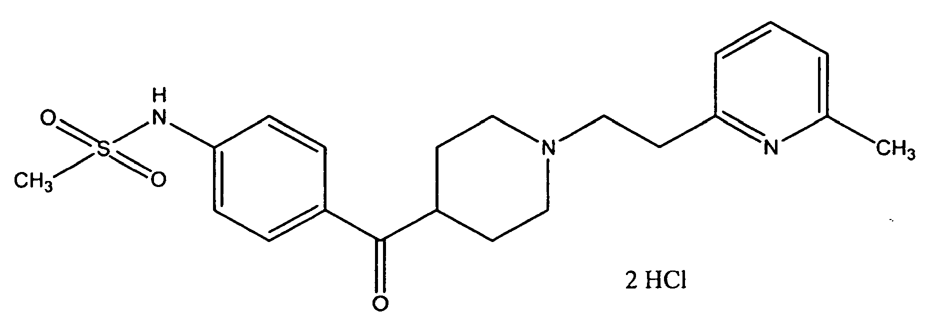 zur Herstellung eines Arzneimittels zur Behandlung eines colorektalen Karzinoms, wobei durch die selektive Blockade eines HERG-Kaliumkanals als eines funktionellen Tumormarkers für das colorektale Karzinom durch das Antiarrhythmikum E-4031 die Karzinomzellen zum Absterben gelangen.

## Claims

1. A method for diagnosing a colorectal carcinoma, **characterized in that** at least one HERG potassium channel is detected as a selective tumor marker for colorectal carcinoma in a tissue sample of the human large intestine or rectum or in lymph nodes or in body fluids or in the stool.

2. A use of the antiarrhythmic drug 4(1-(2-(6-methyl-2-pyridinyl)ethyl-4-piperidinyl)carbonyl)methane-sulfoanilide · 2HCl (E-4031) of formula I 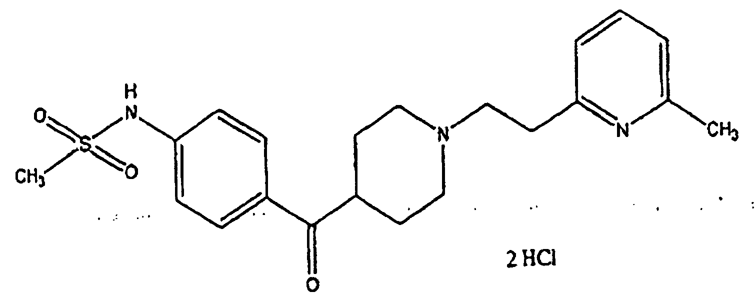 for producing a pharmaceutical drug for treatment of a colorectal carcinoma, wherein by selective blockade of a HERG potassium channel as a functional tumor marker for colorectal cancer, the carcinoma cells die off due to the antiarrhythmic drug E-4031.

## Revendications

1. Procédé de diagnostic d'un carcinome colorectal, **caractérisé en ce qu'**on met en évidence dans un échantillon de tissu du gros intestin ou du rectum humain ou dans le ganglion lymphatique ou dans les liquides physiologiques ou dans les selles au moins un canal à potassium HERG servant de marqueur tumoral sélectif pour le carcinome colorectal.

2. Utilisation de l'antiarrhythmique 4-[1-[2-(6-méthyle-2-pyridinyle)éthyle-4-pipéridinyle]carbonyle]méthane-sulfoanilide, 2HCI(E-4031) de la formule I 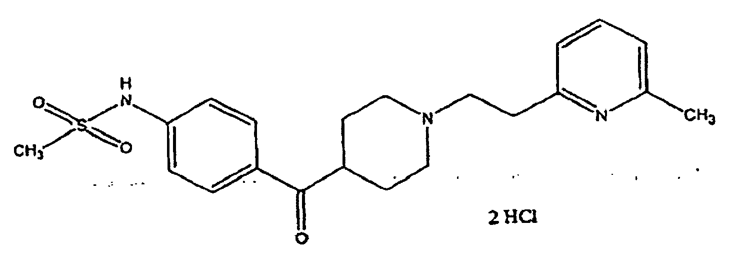 pour la préparation d'un médicament pour le traitement d'un carcinome colorectal, sachant que les cellules de carcinome arrivent à être détruites par le blocage sélectif d'un canal à potassium HERG servant de marqueur tumoral fonctionnel pour le carcinome colorectal grâce à l'antiarrythmique E-4031.
